# EUROPÄISCHE PATENTSCHRIFT

(11) **EP 3 679 351 B1**
(45) Veröffentlichungstag und Bekanntmachung des Hinweises auf die Patenterteilung: **02.09.2026**
(21) Anmeldenummer: 18752104.2
(22) Anmeldetag: 27.07.2018
(51) Int. Cl.: G01N 21/57

(54) **VERFAHREN UND VORRICHTUNG ZUM EINLEITEN EINES ONLINE-BESTELLVORGANGS FÜR EIN INDIVIDUELL HERGESTELLTES HAARPFLEGEPRODUKT**
METHOD AND DEVICE FOR INITIATING AN ONLINE ORDERING PROCESS FOR AN INDIVIDUALLY PRODUCED HAIR CARE PRODUCT
PROCÉDÉ ET DISPOSITIF POUR INITIER UNE PROCÉDURE DE COMMANDE EN LIGNE POUR UN PRODUIT DE SOIN CAPILLAIRE PERSONNALISÉ

(30) Priorität: 07.09.2017 DE 102017215791
(43) Veröffentlichungstag der Anmeldung: 15.07.2020
(73) Patentinhaber: Henkel AG & Co. KGaA, 40589 Düsseldorf (DE)
(72) Erfinder: MÜLLER, Burkhard, 40221 Düsseldorf (DE); LECHNER, Torsten, 40764 Langenfeld (DE)
(86) Internationale Anmeldenummer: PCT/EP2018/070378
(87) Internationale Veröffentlichungsnummer: WO 2019/048128

(56) Entgegenhaltungen:
- WO-A1-2017/004008
- CN-A- 103 063 617
- DE-A1- 102016 225 676
- DE-B3- 102007 055 100
- JP-A- 2000 206 044
- N LEFAUDEUX ET AL: "New luster formula for the characterization of hair tresses using polarization imaging", JOURNAL OF COSMETIC SCIENCE, 1 March 2009 (2009-03-01), United States, pages 153 - 169, XP055147578, Retrieved from the Internet <URL:http://www.ncbi.nlm.nih.gov/pubmed/19450417> DOI: 10.1111/j.1468-2494.2010.00534_7.x
- MCMULLEN R; JACHOWICZ J.: "Optical properties of hair: effect of treatments on luster as quantified by image analysis.", JOURNAL OF COSMETIC SCIENCE, vol. 54, no. 4, 1 July 2003 (2003-07-01), US, pages 335 - 351, XP055504308, ISSN: 1525-7886
- KAPLAN ET AL: "The shine problem in hair: Review of imaging methods and measures for luster", vol. 60, no. 2, 1 March 2009 (2009-03-01), pages 111 - 123, XP009503908, ISSN: 1525-7886, Retrieved from the Internet <URL:http://journal.scconline.org/contents/cc2009/cc060n02.html>

## Beschreibung

### Geänderte Beschreibung

Die Erfindung betrifft ein Verfahren zum Einleiten eines Online-Bestellvorgangs für ein individuell hergestelltes Haarpflegeprodukt gemäß Anspruch 1 und eine Integrierte tragbare Vorrichtung ausgebildet zum Ermitteln eines Glanzwerts von Haaren, zum Ermitteln eines Haarpflegeprodukts zur Glanzerhöhung und zum Einleiten eines Online-Bestellvorgangs für ein individuell hergestelltes Haarpflegeprodukt gemäß Anspruch 8.

Glänzendes, gesund aussehendes Haar kann ein wichtiges kosmetisches Ziel sein, welches mittels kosmetischer Produkte zur Glanzerhöhung erzielbar sein kann.

Eine Eignung eines Glanzerhöhungsprodukts für einen Haartyp kann davon abhängig sein, ob das Haar bereits stark glänzt oder nicht, anders ausgedrückt von einem Glanzwert des Haars.

Nutzer wissen oft nicht, welche Glanzerhöhungsprodukte für ihren Haartyp (z.B. ihren gegenwärtigen Haarglanzwert) am besten geeignet sind, um glänzende(re)s Haar zu erzeugen. Ein Grund dafür kann sein, dass ihnen keine Möglichkeit zur Verfügung steht, um objektiv ihren Haarglanzwert zu beurteilen. Mitunter wird zur Unterscheidung des Haarglanzwerts vor einer Anwendung eines Haarglanzprodukts und einem Haarglanzwert nach der Anwendung des Haarglanzprodukts der Haarglanzwert vor der Anwendung auch als ursprünglicher Haarglanzwert, Anfangs-Haarglanzwert, oder Ausgangs-Haarglanzwert bezeichnet.

Ein weiterer Grund dafür, dass Nutzer oft nicht wissen, welche Glanzerhöhungsprodukte für sie geeignet sind, kann sein, dass die Produkte nicht mit Informationen dahingehend versehen sind, für welchen Anfangs-Haarglanzwert sie geeignet sind.

In den wissenschaftlichen Veröffentlichungen "New Luster Formula for the Characterization of Hair Tresses using Polarization Imaging" von N. Lefaudeux et al., Journal of cosmetic science Seiten 153-169, "Optical Properties of Hair: Effect of treatments on Luster as Quantified by Image Analysis" von Mcmullen R. et al., Journal of Cosmetic Science, bd. 54, Nr. 4, Seiten 335-351 und "The Shine Problem in Hair: Review of Imaging Methods and Measures for Luster", von Kaplan et al., Journal of Cosmetic Science, Bd. 60, Nr. 2, Seiten 111-123 werden Beispiele für Methoden zur Messung des Glanzwertes von Haaren auf Grundlage von Reflexionsmessungen unter Zuhilfenahme von zylindrischen Trägern beschrieben. Die Dokumente DE102007055100 B3, JP2000206044 A, CN103063617 A, WO2017004008 A1 enthalten weitere Beispiele für Geräte, die zur Messung des Glanzwertes von Haaren geeignet sind. Das Dokument DE102016225676 A1 beschreibt ein Verfahren zur Bestimmung eines Produktes zur Verbesserung des Glanzes von Haaren auf der Grundlage eines bestimmten Porositätsgrades der Haare, wobei Porositätsmessungen mittels Durchleuchten einer Haarprobe durchgeführt werden und daraufhin eine individuelle Empfehlung von Haarpflegeprodukten zur Glanzerhöhung ausgegeben werden kann, sowie das optionale Einleiten eines Bestellvorgangs durch Aufrufen einer Internetseite eines Herstellers von individuellen Haarpflegeprodukten.

Es besteht somit ein Bedarf an einer quantitativen Bewertung eines Glanzes von Haar. Wünschenswert wäre dabei ferner eine einfache Anwendbarkeit ohne oder mit nur geringem apparativem Aufwand, so dass der Haarglanzwert beispielsweise auch von einem Nutzer ermittelt werden kann.

Ferner besteht ein Bedarf daran, eine Produktempfehlung für ein Glanzerhöhungsprodukt zu erhalten, welches für einen Anfangs-Haarglanzwert eines Nutzers geeignet ist.

Erfindungsgemäß werden eine Vorrichtung und ein Verfahren zum Ermitteln eines Glanzwerts von Haaren mithilfe einer externen, bevorzugt mobilen Lichtquelle bereitgestellt, die ein Bild von Haaren aufnimmt, bei denen die Haare in einem definierten Radius um einen zylinderförmigen Gegenstand (zum Beispiel einen Wickler) gekrümmt sein können.

Je nach Haarlänge kann dafür ggf. bereits ein Haar ausreichend sein. Üblicherweise können mindestens etwa 5 Haare, vorzugsweise mindestens etwa 10 Haare, genutzt werden.

In verschiedenen nicht erfindungsgemäßen Beispielen können am Kopf anliegende Haare eine ausreichende Krümmung zum Anwenden des Verfahrens aufweisen, sodass auf einen Gegenstand zum aufwickeln der Haare verzichtet werden kann.

In verschiedenen Ausführungsbeispielen kann als der zylinderförmige Gegenstand ein möglichst exakt kreisförmiger Gegenstand genutzt werden. D.h., dass ein Verhältnis von größtem und kleinstem Radius des Zylinders möglichst nah an eins ist.

Ein Ermitteln des Glanzwerts kann in verschiedenen Beispielen mittels herkömmlicher Verfahren durchgeführt werden.

Erfindungsgemäß wird das Haar mit Licht beleuchtet.

Da das Haar auf dem Zylinder angeordnet ist, können sich unterschiedliche Winkel zwischen Lichtquelle und Haaroberfläche ergeben, und ferner winkelabhängige Reflexions- oder Streuungsintensitäten.

Während des Beleuchtens des Haars mit Licht wird erfindungsgemäß ein digitales Bild von dem Haar aufgenommen.

In verschiedenen Ausführungsbeispielen kann ein Verfahren zum Ermitteln des Glanzwerts genutzt werden, welches an ein Verfahren von Reich-Robbins angelehnt sein kann. Dabei kann in dem digitalen Bild ein Anteil diffuser Streuung näherungsweise aus einer Signalintensität bei Winkeln nahe 45° ermittelt und von einem Gesamtsignal subtrahiert werden. Der Glanz ergibt sich aus einem Verhältnis von Glanzreflexion und Streuung sowie einer Glanzbandbreite.

In verschiedenen Ausführungsbeispielen kann ein Verfahren zum Ermitteln des Glanzwerts nach Lefaudeux et al. ("New luster formula for the characterization of hair tresses using polarization imaging", Third Annular Conference on applied Hair Science, Sep 2008) verwendet werden, oder eine von der Firma Bossa Nova genutzte Technologie (http://www.bossanovatech.com/samba_hair_system_technology.htm).

Das Licht, mit welchem das Haar beleuchtet wird, kann in verschiedenen Ausführungsbeispielen polarisiertes Licht sein.

Erfindungsgemäß ist die Kamera, mittels welcher das digitale Bild aufgenommen wird, eine Digitalkamera eines Smartphones, Tablets oder Phablets.

Erfindungsgemäß ist die Lichtquelle, mittels welcher das Haar beleuchtet wird, eine Lichtquelle eines Smartphones, Tablets oder Phablets. Somit kann in verschiedenen Ausführungsbeispielen das polarisierte Licht bereitgestellt werden mittels eines

Aufsatzes für das Smartphone, Tablet, usw., zum Beispiel ein CPL-Polfilter-Aufsatz der Firma Memteq.

In verschiedenen Ausführungsbeispielen kann mittels der letztgenannten Verfahren eine Unterscheidung einer Reflexion von einer Vorderseite von einer Reflexion von einer Rückseite des Haares ermöglicht sein.

In verschiedenen Ausführungsbeispielen können Kalibrationsmessungen an Haaren mit bekannten Glanzwerten ausgeführt werden. Ergebnisse der Kalibrationsmessungen können in verschiedenen Ausführungsbeispielen zu Glanzstufen gruppiert und in eine Datenbank eingetragen werden. Für jede Glanzstufe kann in verschiedenen Ausführungsbeispielen in die Datenbank eine entsprechende Produktempfehlung eingetragen werden.

Bei geringen Glanzstufen können in verschiedenen Ausführungsbeispielen Produkte empfohlen werden, die den Haarglanz stark erhöhen. Bei hohen Glanzstufen können verschiedenen Ausführungsbeispielen Produkte empfohlen werden, die den Haarglanz mäßig erhöhen.

In verschiedenen Ausführungsbeispielen wird ein Verfahren zum Ermitteln eines Glanzwerts von Haaren, der z.B. als Glanzstufe quantifiziert sein kann, bereitgestellt.

Der ermittelte Glanzwert wird erfindungsgemäß genutzt, um ein für Haar mit dem ermittelten Glanzwert empfehlenswertes Produkt zur Glanzerhöhung zu ermitteln.

In verschiedenen Ausführungsbeispielen kann das Ermitteln des Glanzwerts genutzt werden, um einen Erfolg einer Haarbehandlung zur Glanzerhöhung zu ermitteln.

Ein digitales Bild, auf welchem Haare abgebildet sind, wird erfindungsgemäß mittels einer Digitalkamera aufgenommen**.**

Während des Aufnehmens des Bildes werden erfindungsgemäß die Haare, die entlang einer im Wesentlichen zylinderförmigen Fläche gekrümmt sein können, mit Licht, z.B. mit polarisiertem Licht, beleuchtet**.**

In verschiedenen Ausführungsbeispielen kann die im Wesentlichen zylinderförmige Fläche eine Oberfläche eines im Wesentichen zylinderförmigen Gegenstands oder zumindest ein Teil davon sein.

In nicht erfindungsgemäßen Beispielen kann die im Wesentlichen zylinderförmige Fläche eine Kopfoberfläche des Nutzers oder zumindest einen Teil davon sein.

In verschiedenen Ausführungsbeispielen kann das Verfahren mit nur geringem oder ohne apparativen Aufwand ausführbar sein. Beispielsweise kann das Verfahren mittels einer App auf einem Tablet oder einem Smartphone ausführbar sein. Das kann es einem Nutzer beispielsweise ermöglichen, auch ohne professionelle Unterstützung und ohne eine Haarprobe für eine Manipulation im Labor bereitstellen zu müssen, den Glanzwert seines Haars, z.B. seiner Frisur, zu bestimmen, beispielsweise unter Verwendung eines Smartphones oder Tablets zum Aufnehmen eines digitalen Bildes der mit der beschriebenen Krümmung angeordneten Haare und eines Ermittelns des Glättewerts mittels des Smartphones/Tablets, z.B. mittels einer im Smartphone/Tablet bereitgestellten App. In verschiedenen Ausführungsbeispielen kann das Smartphone/Tablet genutzt werden, um das Bild einer externen Datenverarbeitungsvorrichtung, z.B. einer Prozessor-Cloud-Architektur (auch als Cloud bezeichnet), bereitzustellen und die dort ermittelten Ergebnisse zu empfangen und dem Nutzer anzuzeigen.

Ein Nutzer kann in verschiedenen Ausführungsbeispielen eine exakte, auf seine Anfangs-Haarglanz abgestimmte Produktempfehlung, z.B. zur Erhöhung des Haarglanzes, bekommen, ohne eine eigene Haarbeurteilung vornehmen zu müssen, sondern beispielsweise einfach, indem er eines oder mehrere Bilder von seinen Haaren mit seinem Smartphone aufnimmt oder aufnehmen lässt und diese mittels einer App zum Ermitteln eines Haarpflegeprodukts und/oder einer Haarbehandlungsempfehlung auswerten lässt.

Das erfindungsgemäße Verfahren umfasst das Ermitteln eines Glanzwerts von Haaren. Das Verfahren weist ein Beleuchten mindestens eines Haars mit Licht auf, wobei das mindestens eine Haar derart entlang eines zylinderabschnittsähnlichen Trägers verlaufend angeordnet ist, dass eine Mehrzahl von nebeneinander angeordneten Haarabschnitten im Wesentlichen senkrecht zu einer Zylinderachse angeordnet ist, während des Beleuchtens ein Aufnehmen eines digitalen Bildes des mindestens einen Haars mittels einer tragbaren Kamera und ein Ermitteln eines Glanzwert des Haars basierend auf dem digitalen Bild.

In verschiedenen Ausführungsbeispielen kann das Licht linear polarisiert sein, wobei eine Schwingungsebene des polarisierten Lichts entlang der Zylinderachse verlaufen kann.

Erfindungsgemäß weist das Ermitteln des Glanzwerts des Haars ein Ermitteln eines maximalen Helligkeitswerts in einem Haarbereich der digitalen Abbildung und ein Zuordnen des maximalen Helligkeitswerts zu einem Glanzwert auf.

In verschiedenen Ausführungsbeispielen kann das Ermitteln des Glanzwerts des Haars ein Ermitteln eines Glanzbereichs in einem Haarbereich der digitalen Abbildung und ein Summieren von Glanz-Helligkeitswerten aller Bildelemente im Glanzbereich und ein Zuordnen der Summe zu einem Glanzwert aufweisen.

In verschiedenen Ausführungsbeispielen kann das Verfahren ferner ein Anordnen der tragbaren Kamera aufweisen, derart, dass ein Winkel zwischen einer Hauptrichtung, aus der das Licht das Haar beleuchtet, und einer optischen Achse der Kamera einen Winkel von etwa 45° aufweist.

Erfindungsgemäß erfolgt das Beleuchten des mindestens einen Haars mittels einer Lichtquelle, welche zusammen mit der Kamera Teil einer integrierten tragbaren Vorrichtung ist.

In verschiedenen Ausführungsbeispielen kann das Verfahren ferner ein Anordnen des Haars auf einem zylinderartigen Körper aufweisen.

In verschiedenen Ausführungsbeispielen kann das digitale Bild in einem Farbraum parametrisiert sein, der eine Helligkeit als einen Parameter aufweist.

Erfindungsgemäß wird ein Verfahren zum Ermitteln eines Haarpflegeprodukts bereitgestellt. Das Verfahren weist ein Ermitteln eines Glanzwerts von Haaren gemäß der beanspruchten Erfindung und ein Ermitteln eines Haarpflegeprodukts zur Glanzerhöhung basierend auf dem ermittelten Glanzwert auf.

Erfindungsgemäß wird auch eine integrierte tragbare Vorrichtung ausgebildet zum Ermitteln eines Glanzwerts von Haaren, zum Ermitteln eines Haarpflegeprodukts zur Glanzerhöhung und zum Einleiten eines Online-Bestellvorgangs für ein individuell hergestelltes Haarpflegeprodukt gemäß Anspruch 8 bereitgestellt.

In verschiedenen Ausführungsbeispielen kann das von der Lichtquelle abgestrahlte Licht linear polarisiertes Licht sein.

In verschiedenen Ausführungsbeispielen kann der Glanzwert einen Wert aus einer mehrstufigen Glanzwertskala aufweisen, die Glanzwerte von sehr wenig bis sehr stark aufweisen kann, wobei das Haarpflegeprodukt zur Glanzerhöhung eines sein kann aus einer Mehrzahl von Haarpflegeprodukten zur Glanzerhöhung, wobei jedem der Mehrzahl von Haarpflegeprodukten zur Glanzerhöhung eine Glanzerhöhungsstufe zugeordnet ist, wobei das Ermitteln eines Haarpflegeprodukts basierend auf dem ermittelten Glanzwert ein Ermitteln eines Haarpflegeprodukts mit einer höheren Glanzerzeugungsstufe für Haar mit einem niedrigeren Glanzwert aufweist.

In nicht erfindungsgemäßen Beispielen kann das Aufnehmen des mindestens einen digitalen Bildes ein Aufnehmen einer Mehrzahl von digitalen Bildern aufweisen, wobei auf jedem Bild eine Haarfrisur aus einer anderen Blickrichtung dargestellt sein kann.

In nicht erfindungsgemäßen Beispielen kann das Ermitteln des Glanzwerts von Haaren ein Ermitteln eines gemeinsamen Frisur-Glanzwerts basierend auf der Mehrzahl von Glanzwerten aus der Mehrzahl von digitalen Bildern aufweisen.

In verschiedenen Ausführungsbeispielen kann das Ermitteln eines Glanzwerts des Haars basierend auf dem digitalen Bild ein Ermitteln eines Haardarstellungsbereichs, in welchem das mindestens eine Haar des Nutzers dargestellt ist, aufweisen.

In verschiedenen Ausführungsbeispielen kann das Ermitteln eines Glanzwerts des Haars basierend auf dem digitalen Bild ferner ein Festlegen des gesamten Haardarstellungsbereichs als Haaruntersuchungsbereich aufweisen.

In verschiedenen Ausführungsbeispielen kann das Verfahren ferner ein Ausgeben des ermittelten Glanzwerts und/oder des ermittelten Haarpflegeprodukts aufweisen.

In verschiedenen Ausführungsbeispielen kann Ausgeben des ermittelten Glanzwerts und/oder des ermittelten Haarpflegeprodukts ein Darstellen mittels einer Anzeigevorrichtung aufweisen.

Ausführungsbeispiele der beanspruchten Erfindung und andere Beispiele sind in den Figuren dargestellt und werden im Folgenden näher erläutert.

Es zeigen
Figur 1A eine graphische Darstellung einer Vorrichtung zum Ermitteln eines Glanzwerts von Haaren;
Figuren 1B eine graphische Darstellung einer Vorrichtung zum Ermitteln eines Glanzwerts von Haaren gemäß verschiedenen Ausführungsbeispielen, wobei ein Anwenden eines Verfahrens zum Ermitteln eines Glanzwerts von Haaren gemäß verschiedenen Ausführungsbeispielen veranschaulicht ist;
Figuren 2A und 2B jeweils digitale Bilder, die mittels einer Vorrichtung zum Ermitteln eines Glanzwerts von Haaren gemäß verschiedenen Ausführungsbeispielen oder beim Ausführen eines Verfahrens zum Ermitteln eines Glanzwerts von Haaren gemäß verschiedenen Ausführungsbeispielen aufgenommen wurden;
Figur 3 einen Helligkeitsverlauf, welcher beim Anwenden eines Verfahrens zum Ermitteln eines Glanzwerts von Haaren gemäß verschiedenen Ausführungsbeispielen ermittelt wurde;
Figur 4 ein Flussdiagramm eines Verfahrens zum Ermitteln eines Glanzwerts von Haaren gemäß verschiedenen Ausführungsbeispielen;
Figur 5 ein Flussdiagramm eines Verfahrens zum Ermitteln eines Haarpflegeprodukts und/oder einer Haarbehandlungsempfehlung gemäß verschiedenen Ausführungsbeispielen.

In der folgenden ausführlichen Beschreibung wird auf die beigefügten Zeichnungen Bezug genommen, die Teil der vorliegenden Anmeldung bilden und in denen zur Veranschaulichung spezifische Ausführungsformen gezeigt sind, in denen die Erfindung ausgeübt werden kann. In dieser Hinsicht wird Richtungsterminologie wie etwa "oben", "unten", "vorne", "hinten", "vorderes", "hinteres", usw. mit Bezug auf die Orientierung der beschriebenen Figur(en) verwendet. Da Komponenten von Ausführungsformen in einer Anzahl verschiedener Orientierungen positioniert werden können, dient die Richtungsterminologie zur Veranschaulichung und ist auf keinerlei Weise einschränkend. Es versteht sich, dass andere Ausführungsformen benutzt und strukturelle oder logische Änderungen vorgenommen werden können, ohne von dem Schutzumfang der vorliegenden Erfindung abzuweichen. Die folgende ausführliche Beschreibung ist deshalb nicht in einschränkendem Sinne aufzufassen, und der Schutzumfang der vorliegenden Erfindung wird durch die angefügten Ansprüche definiert.

Unter einem digitalen Bild kann hierin ein Datenpaket verstanden werden, welches von einem Datenverarbeitungssystem als zweidimensionale (flächige) Anordnung von Bildpunkten (auch als Pixel bezeichnet) darstellbar ist, beispielsweise in einem Koordinatensystem, welches eine x-Achse und eine y-Achse aufweist, wobei jeder Bildpunkt zumindest eine Bildposition als x,y-Koordinatenpaar und eine Intensitätsinformation aufweist, wobei die Intensitätsinformation beispielsweise als Farbe eines Pixels eines Monitors oder eines gedruckten Punktes eines ausgedruckten Bilds darstellbar ist. Die Intensitätsinformation kann sich bei einem Farbbild auf einzelne Farbkanäle beziehen. Das digitale Bild kann beispielsweise ein mit einer Digitalkamera aufgenommenes Foto oder ein Einzelbild einer mit einer digitalen Kamera aufgenommenen Videosequenz sein.

Bei einem hierin genannten Smartphone ist dies, sofern aus dem Kontext nicht etwas Anderes hervorgeht, als stellvertretend zu verstehen für alle ähnlichen Arten von tragbarer Datenverarbeitungsvorrichtung, d.h. Smartphones, Tablets, Phablets, Laptops, usw. Sinngemäßes gilt für Smartphonekameras und Ähnliches.

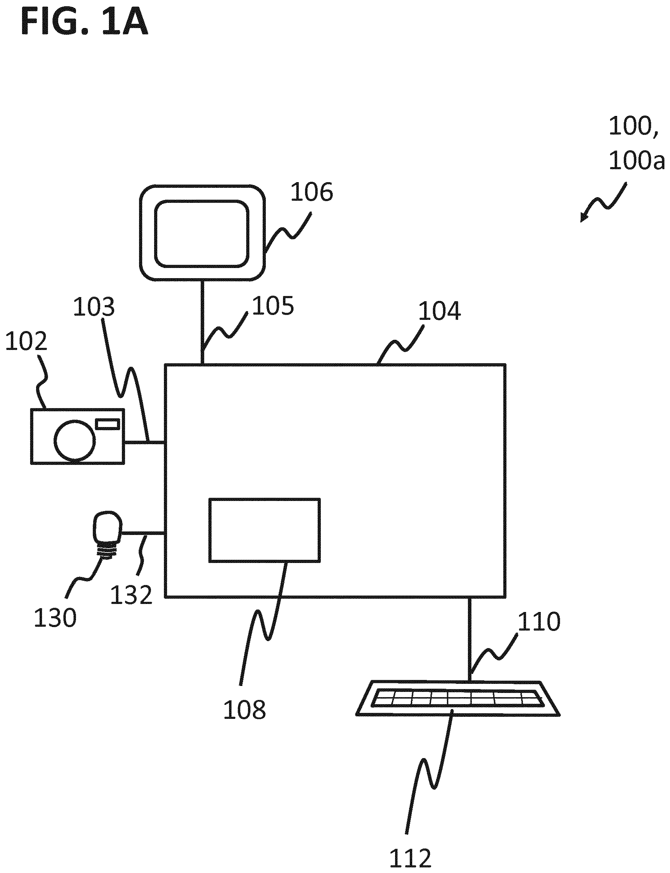

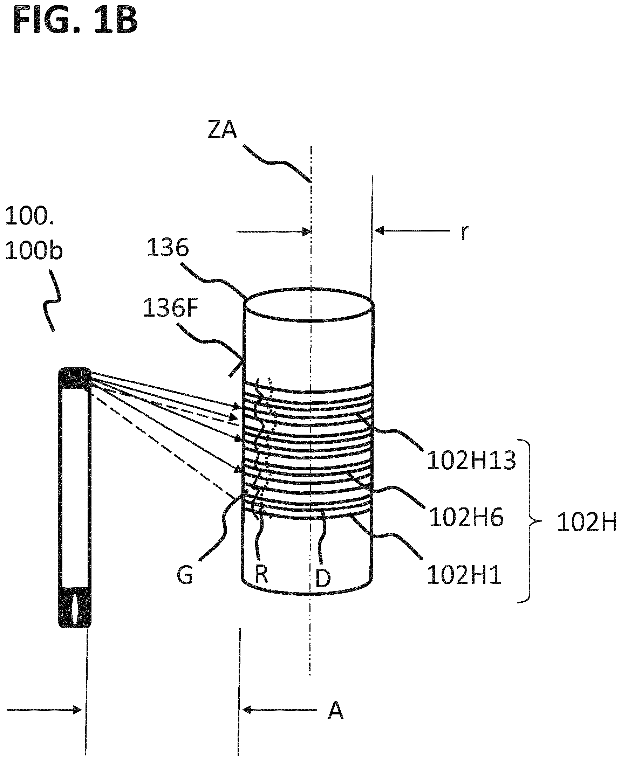

FIG. 1A zeigt eine graphische Darstellung einer Vorrichtung 100, 100a zum Ermitteln eines Glanzwerts von Haaren, und FIG. 1B zeigt eine graphische Darstellung einer Vorrichtung 100, 100b zum Ermitteln eines Glanzwerts von Haaren gemäß verschiedenen Ausführungsbeispielen, wobei ein Anwenden eines Verfahrens zum Ermitteln eines Glanzwerts von Haaren gemäß verschiedenen Ausführungsbeispielen veranschaulicht ist. Die Vorrichtung 100 ist zum Ermitteln eines Haarpflegeprodukts und einer optionalen Haarbehandlungsempfehlung gemäß verschiedenen Ausführungsbeispielen.

Erfindungsgemäß weist die Vorrichtung 100 zum Ermitteln eines Glanzwerts von Haaren und zum Ermitteln eines Haarpflegeprodukts eine tragbare Datenverarbeitungsvorrichtung 104 auf.

Die tragbare Datenverarbeitungsvorrichtung 104 kann beispielsweise einen ein Tablet, ein Smartphone aufweisen.

Die tragbare Datenverarbeitungsvorrichtung 104 ist eingerichtet ein Verfahren zum Ermitteln eines Glanzwerts von Haaren und zum Ermitteln eines Haarpflegeprodukts gemäß verschiedenen Ausführungsbeispielen auszuführen. Die Datenverarbeitungsvorrichtung 104 kann einen Prozessor 108, beispielsweise einen Mikroprozessor, aufweisen.

In verschiedenen Ausführungsbeispielen kann die Vorrichtung 100 zum Ermitteln eines Glanzwerts von Haaren und zum Ermitteln eines Haarpflegeprodukts eine Ausgabevorrichtung 106, z.B. eine Anzeigevorrichtung, aufweisen. Anstelle der Anzeigevorrichtung oder zusätzlich zur Anzeigevorrichtung kann in verschiedenen Ausführungsbeispielen auch eine andere Art von Ausgabevorrichtung, z.B. ein Lautsprecher, bereitgestellt sein.

Die Ausgabevorrichtung 106, z.B. eine Anzeigevorrichtung, kann beispielsweise einen (z.B. berührungsempfindlichen) Bildschirm eines Smartphones, eines Laptops oder eines Phablets aufweisen. Die Ausgabevorrichtung 106, z.B. eine Anzeigevorrichtung, kann genutzt werden, um Ergebnisse des Verfahrens zum Ermitteln eines Glanzwerts von Haaren oder zum Ermitteln eines Haarpflegeprodukts und/oder einer Haarbehandlungsempfehlung darzustellen, z.B. den ermittelten Glanzwert und/oder das ermittelte Pflegeprodukt, Eingabeparameter für das Ausführen des Verfahrens zu erfragen, oder Ähnliches.

Die Ausgabevorrichtung 106, z.B. eine Anzeigevorrichtung, kann mittels einer ersten Datenverbindung 105 mit der Datenverarbeitungsvorrichtung 104 verbunden sein. Die Ausgabevorrichtung 106, z.B. eine Anzeigevorrichtung, kann mit der Datenverarbeitungsvorrichtung 104 mittels der ersten Datenverbindung 105 Daten austauschen. Da erfindungsgemäß die Vorrichtung 100 ein Smartphone, einen Tablet o.ä. aufweist, können die Ausgabevorrichtung 106, z.B. eine Anzeigevorrichtung, und die erste Datenverbindung 105 in der Vorrichtung 100 integriert sein, wie beispielsweise in FIG. 1B für die Vorrichtung 100b dargestellt.

Erfindungsgemäß weist die Vorrichtung 100 zum Ermitteln eines Glanzwerts von Haaren und zum Ermitteln eines Haarpflegeprodukts eine Kamera 102 auf.

Die Kamera 102 ist eingerichtet, ein digitales Bild 200 von Haar 102, z.B. von Haar eines Nutzers, aufzunehmen, beispielsweise wie an anderer Stelle beschrieben.

Die eine Kamera 102 weist erfindungsgemäß eine digitale Fotokamera eines Smartphones, Tablets etc. auf, so dass die Kamera in der Vorrichtung 100 integriert ist, wie beispielsweise in FIG. 1B für die Vorrichtung 100b dargestellt.

In verschiedenen Ausführungsbeispielen kann die Vorrichtung 100 zum Ermitteln eines Glanzwerts von Haaren und zum Ermitteln eines Haarpflegeprodukts und einer optionalen Haarbehandlungsempfehlung eine zweite Datenverbindung 103 zwischen dem Computer 104 und der Kamera 102 aufweisen. Mittels der zweiten Datenverbindung 103 können Daten vom Computer 104 an die Kamera 102 übermittelt werden, beispielsweise für eine, z.B. herkömmliche, Softwaresteuerung der Kamera 102. Ferner können mittels der zweiten Datenverbindung 103

Daten, beispielsweise das/die von der Kamera 102 aufgenommenen digital/en Bild/er, an den Computer 104 übertragen werden. Da erfindungsgemäß die Vorrichtung 100 ein Smartphone, einen Tablet o.ä. aufweist, können die Kamera 102 und die zweite Datenverbindung 103 in der Vorrichtung 100 integriert sein.

Die Datenverarbeitungsvorrichtung 104 kann eingerichtet sein, beispielsweise unter Verwendung des Prozessors 108, das von der Kamera 102 empfangene Bild mittels einer Bildverarbeitungssoftware zu verarbeiten, beispielsweise um auf bekannte Weise in dem empfangenen Bild den Haardarstellungsbereich zu ermitteln und, wie hierin an anderer Stelle für verschiedenen Ausführungsbeispiele beschrieben, den Glanzwert von Haaren mittels im Wesentlichen bekannter Verfahren, z.B. mittels eines Reich-Robbins-Verfahrens oder z.B. mittels eines Verfahrens nach Lefaudeux et al., zu ermitteln. Die Bildverarbeitungssoftware kann in verschiedenen Ausführungsbeispielen eine App aufweisen.

In verschiedenen Ausführungsbeispielen kann die Vorrichtung 100 eine Eingabevorrichtung 112 zum Bereitstellen von Informationen an die Datenverarbeitungsvorrichtung 104 aufweisen, beispielsweise eine berührungsempfindliche Oberfläche der Ausgabevorrichtung 106, eine Tastatur, eine Maus oder ähnliches.

Die Eingabevorrichtung 112 kann mittels einer dritten Datenverbindung 110 mit der Datenverarbeitungsvorrichtung 104 verbunden sein. Die Eingabevorrichtung 112 kann mit der Datenverarbeitungsvorrichtung 104 mittels der dritten Datenverbindung 110 Daten austauschen.

Da erfindungsgemäß die Vorrichtung 100 ein Smartphone, einen Tablet o.ä. aufweist, können die Eingabevorrichtung 112 und die dritte Datenverbindung 110 in der Vorrichtung 100 integriert sein. Die Vorrichtung 100 weist erfindungsgemäß eine Lichtquelle 130 auf.

Die Lichtquelle weist erfindungsgemäß eine integrierte Lampe/Lichtquelle (z.B. LED) eines Smartphones, Tablets o.Ä. auf, oder in nicht erfindungsgemäßen Beispielen eine externe Lichtquelle, welche mittels der Datenverarbeitungsvorrichtung 104 mittels einer Datenverbindung 132 steuerbar sein kann. In verschiedenen Ausführungsbeispielen kann die Lichtquelle ohne Steuerung durch die Datenverarbeitungsvorrichtung (z.B. von Hand) zum Beleuchten der Haare 102H während des Aufnehmens des Bildes angeschaltet werden.

In verschiedenen Ausführungsbeispielen kann mittels der Lichtquelle 130 linear polarisiertes Licht abgestrahlt werden. Im Fall einer Smartphonelampe oder einer sonstigen Lampe, welche eigentlich kein linear polarisiertes Licht abstrahlt, kann eine Zusatzvorrichtung, z.B. ein Aufsatz (z.B. wie oben beschrieben), genutzt werden, um das Licht linear polarisieren.

FIG. 1B zeigt ein Anwendungsbeispiel, bei welchem die Vorrichtung 100, 100b genutzt wird zum Ausführen des Verfahrens zum Ermitteln eines Glanzwerts von Haaren gemäß verschiedenen Ausführungsbeispielen.

Die Vorrichtung 100b weist als integrierte Vorrichtung 100b (beispielhaft ist ein Smartphone dargestellt, stattdessen könnte ein Tablet, Phablet oder Ähnliches verwendet werden) die Datenverarbeitungsvorrichtung 104 mit dem Prozessor 108, die Kamera 102, die Anzeigevorrichtung 106, die Eingabevorrichtung 112 und die Lichtquelle 130 auf.

Damit beim beleuchteten Haar 102H ein Entstehen eines auswertbaren Glanzeffekts bewirkt werden kann, kann es nötig sein, das Haar 102H so anzuordnen, dass einzelne Haare oder zumindest Haarabschnitte 102H1, ..., 102H6, ..., 102H13, ... (wie in FIG. 1B dargestellt) zueinander im Wesentlichen parallel und mit einer Krümmung angeordnet sind.

In FIG. 1B ist dies bewirkt durch Wickelns mindestens eines Haars 102H um einen zylinderartigen Gegenstand 136, z.B. einen Wickler. Bei Verwenden des zylinderartigen Gegenstands kann ein Kreiszylinder genutzt werden, d.h. ein Zylinder mit kreisförmiger Grundfläche. Alternativ kann ein Zylinder mit einer elliptischen Grundfläche genutzt werden, beispielsweise mit einer Grundfläche, bei welcher ein Verhältnis eines größten Radius zu einem kleinsten Radius des Zylinders in einem Bereich von etwa 1,01 bis etwa 1,3 liegt.

Ein Verwenden eines standardisierten zylinderartigen Gegenstands, beispielsweise mit einer definierten Größe und Krümmungsradius (und damit einer gekrümmten Fläche 103F, auf welcher die Haare 102H angeordnet sind, und einer gerade verlaufenden Zylinderachse ZA), kann in verschiedenen Ausführungsbeispielen zu einheitlicheren Absolutwerten von anhand des aufgenommenen Bildes zu ermittelnden Helligkeitswerten führen.

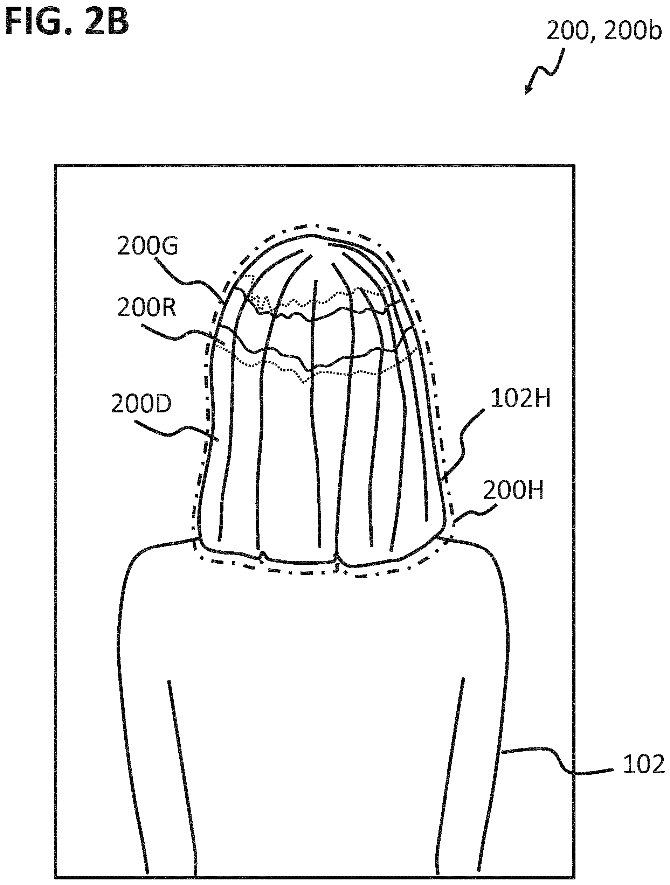

Ein erleichtertes Durchführen des Verfahrens kann in verschiedenen Ausführungsbeispielen ein Verwenden nicht-standardisierter gekrümmter Flächen 136F aufweisen, beispielsweise, wie in FIG. 2B dargestellt, einer gekrümmten Kopfoberfläche. Dabei kann die "Zylinderachse" oder alternativ ein der Lichtquelle zugewandter Scheitelpunkt des Krümmungsradius entlang einer gekrümmten Linie verlaufen, statt wie beim zylinderförmigen Körper 136 exakt gerade zu verlaufen.

Zum Erzeugen der Haaranordnung aus FIG. 1B können beispielsweise dem Nutzer 102 Haare 102H abgeschnitten worden sein, z.B. zwischen etwa 5 und etwa 1000 Haaren 102H, z.B. zwischen etwa 10 und etwa 50, z.B. etwa 30. Alternativ können die Haare 102H zum Weiteren Ausführen des Verfahrens, sofern sie lang genug sind, am Kopf belassen werden.

Die Haare 102H können auf dem (z.B. exakt kreisrunden) Zylinder (z.B. einem Kunststoffzylinder) so angeordnet werden, dass sie alle gestreckt oder im Wesentlichen gestreckt, exakt oder im Wesentlichen parallel zueinander liegen.

Mittels der Vorrichtung 100b (z.B. dem Smartphone 100b) können die Haare 102H angestrahlt und gleichzeitig ein Foto genommen werden.

Für eine Auswertung kann im Beispiel ein Kreisbogen von 90° des Zylinders herangezogen werden.

Die beschriebene Anordnung und Durchführung kann in verschiedenen Ausführungsbeispielen zu einer winkelabhängigen Lichtreflexion führen, welche reflektiertes und gestreutes Licht aufweist, z.B. daraus besteht.

In verschiedenen Ausführungsbeispielen kann das Ermitteln des Glanzwertes an den Haaren mittels einer im Smartphone installierten Software, z.B. einer App, nach Reich-Robbins oder einem anderen bekannten Verfahren ausgeführt werden.

In verschiedenen Ausführungsbeispielen kann beim Auswerten eine Lichtintensität, mit welcher das Haar 102H beleuchtet wird, einbezogen werden. Dafür kann in verschiedenen Ausführungsbeispielen eine Leuchtstärke der Lichtquelle einbezogen werden, beispielsweise anhand bekannter technischer Daten der Lichtquelle 130.

In verschiedenen Ausführungsbeispielen kann ferner ein Abstand A zwischen der Lichtquelle 130 und dem mindestens einen Haar 102H des Nutzers 102 herangezogen werden. Der Abstand A kann beispielsweise vorgegeben sein, anhand des Bildes ermittelt werden (z.B. aus einer scheinbaren Größe des Kopfes oder des zylinderartigen Gegenstands 136) oder mittels einer zusätzlich bereitgestellten (nicht dargestellten) Abstandsmessvorrichtung gemessen werden.

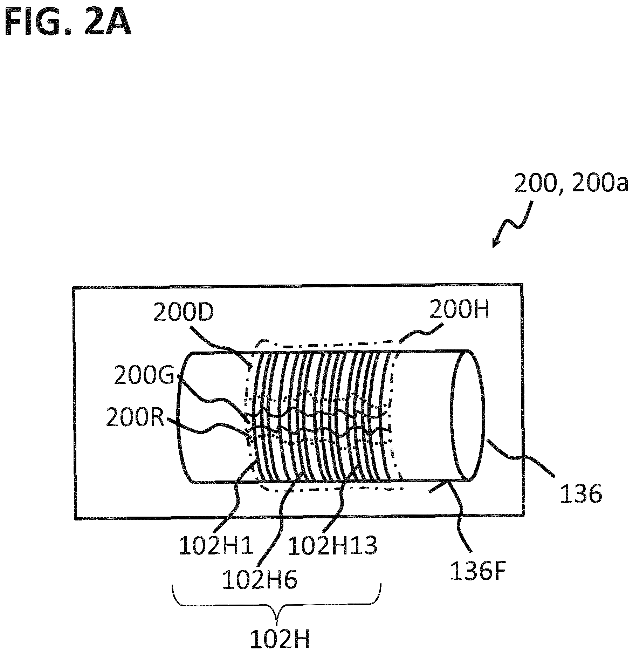

Figuren 2A und 2B zeigen jeweils digitale Bilder 200 (200a oder 200b), die mittels einer Vorrichtung zum Ermitteln eines Glanzwerts von Haaren oder beim Ausführen eines Verfahrens zum Ermitteln eines Glanzwerts von Haaren aufgenommen wurden, beispielsweise mittels einer der Vorrichtungen 100 aus FIG. 1A oder FIG. 1B.

Das Bild 200, in welchem mindestens ein Haar 102 des Nutzers 102 abgebildet ist, ist bei einem Verfahren zum Ermitteln eines Glanzwerts von Haaren gemäß verschiedenen Ausführungsbeispielen aufgenommen worden und zur Ermittlung des Glanzwerts oder eines Haarpflegeprodukts vorgesehen.

In verschiedenen Ausführungsbeispielen kann das digitale Bild 200 z.B. ein so genanntes Schwarzweißbild, sein, das lediglich Helligkeitsinformation aufweist, beispielsweise ein Graustufenbild, bei welchem Weiß einer größten Helligkeit zugeordnet sein kann, und schwarz einer geringsten Helligkeit.

In verschiedenen Ausführungsbeispielen kann das digitale Bild 200 ein Farbbild 200 sein, das in einem Farbraum (z.B. L*a*b*) mit einer Mehrzahl von Parametern codiert ist, wobei einer der Parameter eine Helligkeit sein kann. Für ein Ausführen des hierin beschriebenen Verfahrens, z.B. zum Ermitteln des Glanzwerts, kann eine Untersuchung des Bildes in verschiedenen Ausführungsbeispielen auf den Helligkeitsparameter beschränkt sein oder werden. In verschiedenen Ausführungsbeispielen kann eine Farbe in die Untersuchung einbezogen sein oder werden.

In verschiedenen Ausführungsbeispielen kann das Bild 200 als ein Bild bereitgestellt werden, welches in einem Farbraum parametrisiert ist, der keinen Helligkeitsparameter aufweist, z.B. RGB. Das Bild 200 kann beispielsweise mittels Transformierens in einen Farbraum, der einen Helligkeitsparameter aufweist umgewandelt werden, z.B. L*a*b*. Für eine nachfolgende Untersuchung zum Ermitteln des Glanzwerts von Haaren kann in verschiedenen Ausführungsbeispielen dann lediglich der Helligkeitsparameter herangezogen werden und die weiteren Parameter (z.B. Farbton, Sättigung) ignoriert werden. In verschiedenen Ausführungsbeispielen können die weiteren Parameter (z.B. Farbton, Sättigung) in die Untersuchung einbezogen sein oder werden.

In verschiedenen Ausführungsbeispielen kann in dem Bild 200, unabhängig davon, ob in dem Bild 200 die im nicht erfindungsgemäßen Beispiel am Kopf des Nutzers 102 gekrümmt angeordneten Haare 102H wie in FIG. 2B oder z.B. ein oder mehrere auf einer im Wesentlichen zylinderförmigen Fläche 136F eines im Wesentlichen zylinderförmigen (z.B. zylinderförmigen oder kreisförmigen) Gegenstands 136 erfindungsgemäß angeordnete Haare 102H (welche noch am Kopf des Nutzers 102 angebracht oder davon entfernt sein können) wie in FIG. 1B und 2A dargestellt, sind, ein Haardarstellungsbereich 200H ermittelt werden, in welchem das mindestens eine Haar 102H, z.B. die Haare 102H (z.B. Haupthaar, also beispielsweise ohne Augenbrauen, Bart, usw.) dargestellt ist/sind. In FIG. 2A und FIG. 2B ist der Haardarstellungsbereich 200H zur besseren Erkennbarkeit leicht über den eigentlichen Haardarstellungsbereich 200H hinausgehend dargestellt.

Der Haardarstellungsbereich 200H kann eine Mehrzahl von Bildpunkten des digitalen Bildes 200 aufweisen, welche das Haar 102H abbilden und welche eine zusammenhängende oder aus einer Mehrzahl von Einzelflächen bestehende Fläche bilden können. Eine Ebene, in welcher der Haardarstellungsbereich angeordnet sein kann, kann beispielsweise durch die x-Achse und die y-Achse des digitalen Bildes 200 definiert sein.

In verschiedenen Ausführungsbeispielen kann das digitale Bild 200 zusätzlich zu dem Haardarstellungsbereich 200H noch weitere Darstellungsbereiche aufweisen, in welchen beispielsweise Objekte, Körperteile, usw. dargestellt sein können.

In verschiedenen Ausführungsbeispielen kann ein Untersuchen des digitalen Bildes 200 zum Ermitteln des Glanzwerts, z.B. hinsichtlich Helligkeitswerten, auf einen Haaruntersuchungsbereich beschränkt sein, statt sich auf einen gesamten Bildbereich zu erstrecken. Der Haaruntersuchungsbereich kann in verschiedenen Ausführungsbeispielen so gewählt sein, dass kein Teil der sonstigen Darstellungsbereiche in den Haaruntersuchungsbereich fällt, also beispielsweise auf den Haardarstellungsbereich 200H beschränkt sein.

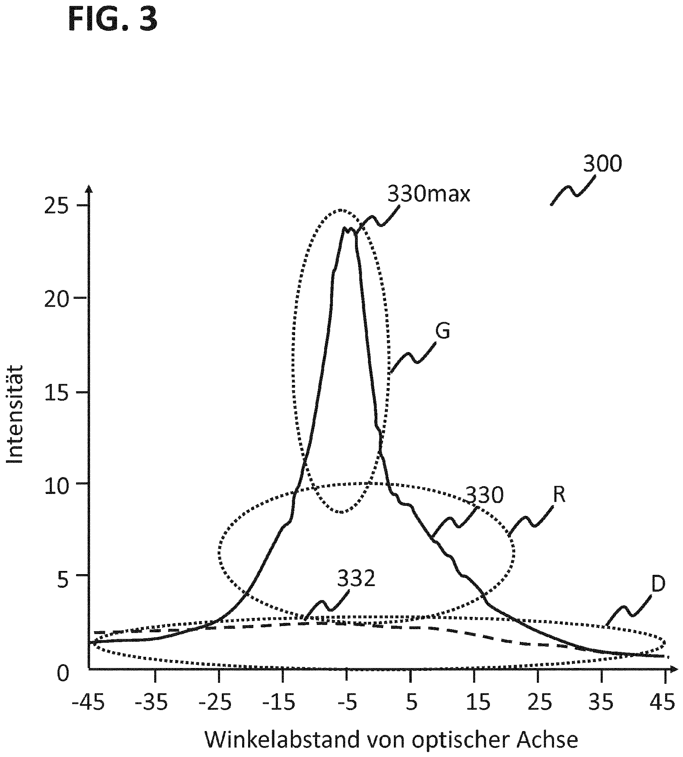

In dem Bild 200 können in gekrümmten Bereichen des Haars 102H unterschiedlich helle Bereiche 200G, 200R und 200D ermittelt werden, in welchen das Licht jeweils unterschiedlich mit dem Haar 102H gewechselwirkt hat: Im Glanzbereich 200G wurde es hauptsächlich von einer Vorderseite des Haars 102H reflektiert, im Randbereich 200R von einer Rückseite des Haars 102H (nach einem Hindurchtreten durch das Haar), und in einem Diffus-Bereich 200D im Wesentlichen gestreut. Entsprechende Lichtanteile sind in FIG. 3 mit G, R und D bezeichnet.

Ein Verhältnis von Intensitäten der reflektierten Anteile G und R zum diffusen Anteil D kann in verschiedenen Ausführungsbeispielen auf bekannte Weise (wie an anderer Stelle hierin genannt) genutzt werden, um den Glanzwert zu ermitteln.

FIG. 3 zeigt einen Helligkeitsverlauf 300, welcher beim Anwenden eines Verfahrens zum Ermitteln eines Glanzwerts von Haaren gemäß verschiedenen Ausführungsbeispielen ermittelt wurde.

Dabei kann der Verlauf im Wesentlichen entlang einer Richtung, in welcher die Haare 102H oder Haarabschnitte verlaufen, ermittelt werden, beispielsweise als über alle Haare gemittelter Wert oder für einen Teilbereich, d.h. einen Teil der Haare 102H.

Lichtanteile, welche dem von der Vorderseite reflektierten Licht G, dem von der Rückseite der Haare reflektierten Licht R oder dem diffus gestreuten Licht D entsprechen, sind in FIG. 3 mittels elliptischer Bereiche ungefähr markiert.

Für die oben beschriebenen Ermittlungen kann in verschiedenen Ausführungsbeispielen eine Software genutzt werden. Dabei kann jede Software genutzt werden, die eine oben beschriebene Funktionalität bereitstellt. Da erfindungsgemäß zum Ausführen des Verfahrens zum Ermitteln eines Glanzwerts von Haaren ein Smartphone oder ein Tablet genutzt wird, kann die Software als eine App bereitgestellt sein.

Mittels Untersuchens der Helligkeitswerte der dargestellten Haare 102H, z.B. mittels Ermittelns einer Mehrzahl von Helligkeitswerten der Haare 102H und einem Auswerten der Helligkeitswerte, z.B. mittels der oben genannten Verfahren, ein Glanzwert für die dargestellten Haare 102H ermittelt werden.

Mittels Berechnens und/oder im Vergleich mit bereitgestellten Referenzwerten, kann ein absoluter Glanzwert für die Haare 102H ermittelt werden, z.B. für die Haare 102H in dem Haaruntersuchungsbereich und/oder für eine Frisur.

In verschiedenen Ausführungsbeispielen kann dem Helligkeitsverhältnis ein Glanzwert zugeordnet werden, beispielsweise mittels Vergleichens mit Referenzwerten. Die Referenzwerte können vorab, z.B. mittels empirischer Daten, erstellt worden sein und beispielsweise als Referenzdatenbank für die Datenverarbeitungsvorrichtung 104 abrufbar sein, z.B. in einem Speicher der Datenverarbeitungsvorrichtung 104 gespeichert, oder beispielsweise aus einer Cloud abrufbar.

Die Referenzdaten können beispielsweise eine mehrstufige Glanzwertskala aufweisen, z.B. mit mindestens zwei Glanzwerten (z.B. von "sehr gering" bis "sehr stark"), z.B. drei bis fünf Glanzwerten, welche für jeden der Glanzwerte der mindestens einen Messwert aufweist, der mittels der ermittelten Mehrzahl von verschiedenen Helligkeitswerten ermittelbar ist, z.B. aus dem Helligkeitsverhältnis von reflektierter (G+R) zu diffuser (D) Streuung.

Erfindungsgemäß wird alternativ oder zusätzlich zum Verhältnis aus reflektierter und diffuser Streuung beispielsweise ein maximaler Helligkeitswert herangezogen.

Um eine Schwankungsbreite zu verkleinern kann als der maximale Helligkeitswert beispielsweise ein arithmetischer Mittelwert aus den z.B. höchsten 5% der Helligkeitswerte genutzt werden.

Eine bespielhafte Zuordnung von Helligkeitsverhältnissen zu Glanzwerten ist in der folgenden Tabelle angegeben:

| Verhältnis von Reflexion (G+R) zu diffuser Streuung (D) | Glanzwert |
|---|---|
| (G+R)/D ≤3 | 1 = schwach glänzend |
| 3 < (G+R)/D ≤ 5 | 2 = mäßig glänzend |
| 5 < (G+R)/D (z.B. ≤ 7) | 3 = stark glänzend |

Erfindungsgemäß kann/können das Ermitteln des Haarglanzwerts und/oder das Ermitteln des Haarpflegeprodukts zur Glanzerhöhung direkt mittels der Datenverarbeitungsvorrichtung 104 ausgeführt werden.

In verschiedenen Ausführungsbeispielen kann/können das Ermitteln des Haarglanzwerts und/oder das Ermitteln des Haarpflegeprodukts zur Glanzerhöhung indirekt mittels der Datenverarbeitungsvorrichtung 104 ausgeführt werden, beispielsweise indem die Datenverarbeitungsvorrichtung 104 eingerichtet ist, das digitale Bild und/oder daraus ermittelte (Helligkeits-)Werte einer externen Datenverarbeitungsvorrichtung, z.B. einer Cloud, bereitzustellen, welche eingerichtet sein kann, aus dem digitalen Bild und/oder den daraus ermittelten (Helligkeits-)Werten den Glanzwert zu ermitteln und/oder aus den ermittelten Werten und/oder aus dem Glanzwert das Haarpflegeprodukt zur Glanzerhöhung zu ermitteln und der Vorrichtung 100 bereitzustellen.

In verschiedenen Ausführungsbeispielen können Aufnahmen der Haare des Nutzers 102 aus verschiedenen Richtungen, d.h. von unterschiedlichen Haarbereichen, gemacht werden.

Beim Anfertigen nur einer Aufnahme kann diese in verschiedenen Ausführungsbeispielen die Haare des Nutzers am Hinterkopf abbilden, denn typischerweise weist der Hinterkopf eine geeignete Krümmung auf. Allerdings kann in verschiedenen Ausführungsbeispielen auch ein anderer Haarbereich des Nutzers 102 als der einzige abgebildete Haarbereich verwendet werden, sofern er die geeignete Krümmung aufweist.

In verschiedenen Ausführungsbeispielen können in den Bildern 200 diejenigen Bereiche, in denen keine oder wenig Haare abgebildet sind, z.B. Gesichtsflächen und/oder Hintergrundbereiche, eliminiert werden, z.B. auf bekannte Weise, z.B. mittels Anwendens eines Morphing-Filters. Anders ausgedrückt kann der Haardarstellungsbereich, der überwiegend nur noch Haare enthalten kann, ermittelt werden, und der Bildbereich, der nicht der Haardarstellungsbereich ist, kann verworfen werden.

Erfindungsgemäß kann basierend auf einem wie oben für verschiedene Ausführungsbeispiele beschrieben ermittelten Glanzwert ein Haarpflegeprodukt, z.B. ein für den Glanzwert geeignetes Haarpflegeprodukt, ermittelt werden.

Das Haarpflegeprodukt kann ein Produkt aus einer Gruppe von Haarpflegeprodukten aufweisen, von denen bekannt oder zu erwarten sein kann, dass ihre Eignung zu einer Anwendung bei Haar von einem Anfangs-Glanzwert des Haars abhängen kann, d.h., dass sie jeweils für Haar mit einem gegebenen Glanzwert besonders gut geeignet sein können.

In verschiedenen Ausführungsbeispielen kann die Gruppe von Haarpflegeprodukten z.B.

Haarpflegeprodukte zur Glanzerhöhung von Haaren aufweisen.

Eine Zuordnung von Glanzwerten und geeigneten Haarpflegeprodukten, z.B.

Glanzerhöhungsprodukten, kann beispielsweise als eine vorgegebene Datenbank, die beispielsweise auf empirischen Daten basieren kann, bereitgestellt sein, z.B. in einem Speicher der Datenverarbeitungsvorrichtung und/oder in einer Cloud.

Die geeigneten Haarpflegeprodukte können dabei chemische Produktgruppen und/oder konkrete Produktnamen, unter welchen der Nutzer diese beispielsweise erwerben könnte, aufweisen.

Das Verfahren zum Ermitteln eines Haarpflegeprodukts und/oder einer Haarbehandlungsempfehlung umfasst erfindungsgemäß ferner auch das Einleiten eines online-Bestellvorgangs des ermittelten Haarpflegeprodukts und optional das Bereitstellen eines Hinweises, wo das ermittelte Haarpflegeprodukt erhältlich ist.

Das erfindungsgemäße Verfahren umfasst auch das Einleiten eines Bestellvorgangs von individuell hergestellten Haarpflegeprodukten.

Immer mehr Kunden wünschen sich ein individuell auf ihre Bedürfnisse abgestimmtes Produkt.

Dabei kann es sich um ein speziell für den Kunden hergestelltes Produkt oder ein sogenanntes "mass customized" Produkt handeln. Bei einem "mass customized" Produkt kann durch Variation von wenigen, aus Kundensicht jedoch entscheidenden Merkmalen eines Produkts, eine Individualisierung erreicht werden. Bevorzugt basieren diese "mass customized" Produkte auf dem Konzept der Modularisierung, das heißt, das Produkt kann aus diversen Modulen/Bausteinen individuell zusammengestellt werden.

Zwischen den vielen unterschiedlichen Merkmalen/Inhaltsstoffen eines Produktes bestehen oftmals zahlreiche Abhängigkeiten, die als "Gebote" oder "Verbote" ausgedrückt werden können. Um eine eindeutige Produktdefinition zu erhalten, ist erfindungsgemäß vorgesehen, dass der Bestellvorgang mit Hilfe eines Produktkonfigurators abläuft. Dieser Konfigurator hilft dem Kunden bei der Auswahl der Merkmale/Inhaltsstoffe und weist ihn auf die zulässigen/unzulässigen Merkmalskombinationen hin, wobei letztere dann nicht ausgewählt werden können.

Bei Haarpflegeprodukten umfassen die relevanten Produktmerkmale insbesondere die chemischen Inhaltsstoffe der Mittel, die physikalischen Eigenschaften der Mittel und die Konfektionsart der Mittel. Mit Hilfe eines Produktkonfigurators wird beispielsweise die Auswahl chemisch und/oder physikalisch inkompatibler Inhaltsstoffe oder die Auswahl für den ermittelten Glanzwert ungeeigneter Inhaltsstoffe vermieden.

Umgekehrt kann die Auswahl für den ermittelten Glanzwert geeigneter Inhaltsstoffe durch den Produktkonfigurator vorgegeben oder vorgeschlagen werden.

Die Haarpflegeprodukte können den Glanzwerten in verschiedenen Stufen zugeordnet sein, oder umgekehrt kann die Gruppe von Haarpflegeprodukten in eine Mehrzahl von Pflegestufen, z.B. Glanzerhöhungsstufen, eingeteilt sein, und dazu können Glanzwerte, für welche die Haarpflegeprodukte der jeweiligen Glanzerhöhungsstufe geeignet sein können, festgelegt sein.

Für Glanzerhöhungsprodukte können für unterschiedliche Glanzerhöhungsstufen beispielsweise Inhaltsstoffe wie z.B. Dimethicone (leichte Glanzerzeugung), Aprikosenkernöl (mittlere Glanzerzeugung) und/oder Phenyltrimethicon (starke Glanzerzeugung) verantwortlich sein. Dementsprechend kann ein Nutzer in verschiedenen Ausführungsbeispielen eine exakte, auf seinen Ausgangs-Haarglanzwert abgestimmte Produktempfehlung, z.B. zur Erhöhung von Haarglanz, bekommen, ohne eine eigene Haarbeurteilung vornehmen zu müssen, sondern beispielsweise indem er, je nach Voraussetzung, z.B. einfach in nicht erfindungsgemäßen Beispielen ohne weitere Vorbereitung (bei glattem, am Kopf anliegenden, im Wesentlichen parallel ausgerichtetem und am Kopf geeignet gekrümmtem Haar), nach einem Anformen der Haare an den Kopf, so dass sie die geeignete Krümmung aufweisen und im Wesentlichen parallel zueinander ausgerichtet sind (bei Haar, welches ohne Manipulation gewellt ist) oder erfindungsgemäß nach einem Anordnen der Haare auf einem im Wesentlichen zylinderförmigen Gegenstand, eines oder mehrere Bilder von seinen Haaren mit seinem Smartphone während eines Beleuchtens der Haare aufnimmt oder aufnehmen lässt und dieses Bild mittels einer App zum Ermitteln eines Haarpflegeprodukts und/oder einer Haarbehandlungsempfehlung auswerten lässt.

In verschiedenen Ausführungsbeispielen kann der Anfangs-Glanzwert, für welchen ein Pflegeprodukt zur Glanzerhöhung geeignet sein kann, einen Wert aus einer, beispielsweise der oben beschriebenen, mehrstufigen Welligkeitsskala aufweisen. Das Haarpflegeprodukt zur Glanzerhöhung kann eines sein aus einer Mehrzahl von Haarpflegeprodukten zur Glanzerhöhung, wobei jedem der Mehrzahl von Haarpflegeprodukten zur Glanzerhöhung eine Glanzerhöhungsstufe zugeordnet sein kann, und wobei das Ermitteln eines Haarpflegeprodukts basierend auf dem ermittelten Glanzwert ein Ermitteln eines Haarpflegeprodukts mit einer höheren Glanzerzeugungsstufe für Haar mit einem niedrigeren Glanzwert aufweisen kann.

In der nachfolgenden Tabelle ist eine beispielhafte Zuordnung von Produktempfehlungen für Haarglanzwerte angegeben:

| Glanzwert | Produktempfehlung |
|---|---|
| 1 = schwach glänzend | Produkt 1 für starke Glanzerhöhung, enthaltend Phenyltrimethicon als stark glanzgebenden Stoff (z.B. mit einem Gewichtsanteil von etwa 1%) |
| 2 = mäßig glänzend | Produkt 2 für starke Glanzerhöhung, enthaltend Aprikosenkernöl als mäßig bis stark glanzgebenden Stoff (z.B. mit einem Gewichtsanteil von etwa 1%) |
| 3 = stark glänzend | Produkt 3 für leichte Glanzerhöhung, enthaltend Dimethicone als leicht glanzgebenden Stoff (z.B. mit einem Gewichtsanteil von etwa 1%) |

In der Tabelle können die Bezeichnungen "Produkt 1" usw. allgemeine Bezeichnungen für die jeweilige Produktgruppe sein, und/oder stellvertretend für jeweils einen oder mehrere Produktnamen, unter welchen der Nutzer das jeweilige Produkt kaufen könnte.

Die Zahl der Glanzstufen, Einstufungen der Produkte usw. sind als beispielhaft zu verstehen. Die Zahl der Glanzstufen kann beispielsweise in einem Bereich von 2 bis etwa 20 liegen, z.B. zwischen 2 und 4, beispielsweise, wie oben beschrieben, 3. Ein Zahlenwert, anhand dessen der Glanzwert zugeordnet wird, kann in verschiedenen Ausführungsbeispielen von dem verwendeten Verfahren zur Glanzermittlung abhängen (z.B. nach Reich-Robbins oder nach Lefaudeux). Üblicherweise kann der Wertebereich sich von etwa 1 bis 20 erstrecken, beispielsweise, wie oben beispielhaft dargestellt, von 1 bis etwa 7.

In verschiedenen Ausführungsbeispielen können alternativ und/oder ergänzend zu den oben angeführten Glanzerhöhungsmitteln andere oder weitere Glanzerhöhungsmittel in die Produktempfehlung einbezogen werden, beispielsweise andere bekannte Glanzerhöhungsmittel, z.B. ein Produkt 1 für eine geringe Glanzerhöhung, enthaltend Trisiloxan (z.B. mit einem Gewichtsanteil von etwa 1%).

Ein Ergebnis des Ermittelns des Haarpflegeprodukts kann dem Nutzer bereitgestellt werden, beispielsweise mittels der Ausgabevorrichtung 106, z.B. angezeigt (z.B. mittels des Smartphone-Displays der Vorrichtung 100, wie beispielhaft in FIG. 1B dargestellt oder einer anderen Anzeigevorrichtung) oder auf andere Weise, beispielsweise mittels Sprachausgabe, zugänglich gemacht werden.

In verschiedenen Ausführungsbeispielen kann das Verfahren zum Ermitteln eines Glanzwerts von Haaren vor und nach einem Anwenden eines Haarpflegemittels zur Glanzerhöhung angewendet werden. Damit kann eine Wirksamkeit des Glanzerhöhungsmittels objektiv ermittelt werden.

In verschiedenen Ausführungsbeispielen kann eine Haarbehandlung zur Glanzerhöhung ermittelt werden. Diese kann beispielsweise Die Empfehlung umfassen, einen Friseur aufzusuchen. In verschiedenen Ausführungsbeispielen kann direkt über die Software/App, welche das Haarpflegeprodukt und/oder die Haarbehandlungsempfehlung ermittelt, ein Buchungsvorgang eingeleitet werden. Dazu können beispielsweise in der Software/App die Kontaktdaten von Friseuren hinterlegt sein und diese dem Nutzer angezeigt werden. Zusätzlich kann über Filter, wie beispielsweise die Postleitzahl, die Auswahl eingeschränkt werden. In verschiedenen Ausführungsbeispielen kann direkt über die Software/App eine Terminbuchung vorgenommen werden. Alternativ kann die Buchung eines Friseurtermins über eine separate Software/App, wie beispielsweise Treatwell, erfolgen.

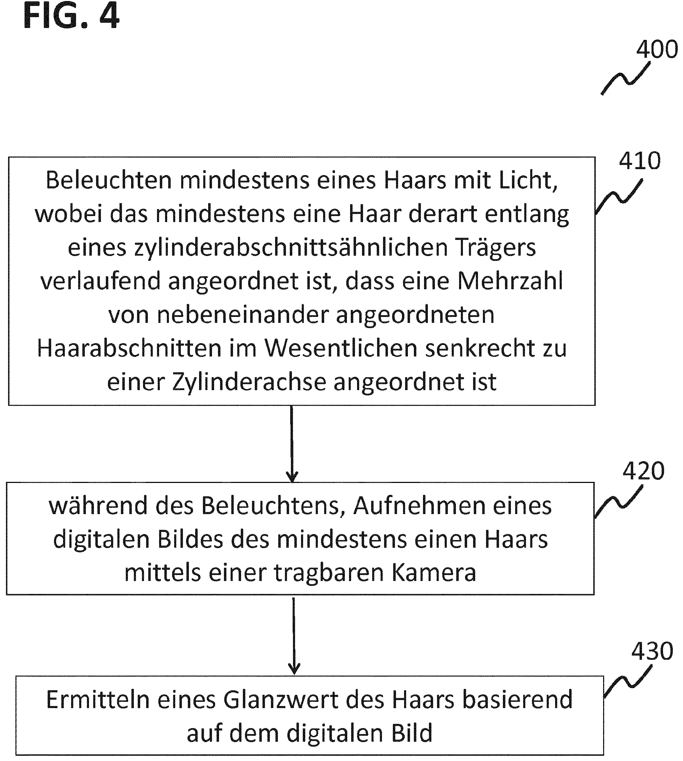

FIG. 4 zeigt ein Flussdiagramm 400 eines Verfahrens zum Ermitteln eines Glanzwerts von Haaren.

Das Verfahren weist ein Beleuchten mindestens eines Haars mit Licht auf, wobei das mindestens eine Haar derart entlang eines zylinderabschnittsähnlichen Trägers verlaufend angeordnet ist, dass eine Mehrzahl von nebeneinander angeordneten Haarabschnitten im Wesentlichen senkrecht zu einer Zylinderachse angeordnet ist (in 410), während des Belichtens, ein Aufnehmen eines digitalen Bildes des mindestens einen Haars mittels einer tragbaren Kamera (in 420), und ein Ermitteln eines Glanzwert des Haars basierend auf dem digitalen Bild (in 430).

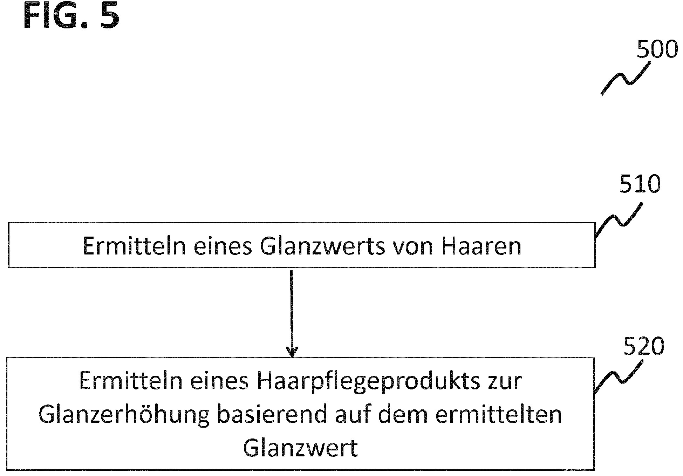

FIG. 5 zeigt ein Flussdiagramm 500 eines Verfahrens zum Ermitteln eines Glanzwerts von Haaren.

Das Verfahren weist ein Ermitteln eines Glanzwerts von Haaren auf (in 510), beispielsweise gemäß verschiedenen oben beschriebenen Ausführungsbeispielen, und ein Ermitteln eines Haarpflegeprodukts und/oder einer Haarbehandlungsempfehlung zur Glanzerhöhung basierend auf dem ermittelten Glanzwert (in 520).

Weitere vorteilhafte Ausgestaltungen des Verfahrens ergeben sich aus der Beschreibung der Vorrichtung und umgekehrt.

## Patentansprüche

1. Verfahren zum Einleiten eines Online-Bestellvorgangs für ein individuell hergestelltes Haarpflegeprodukt, aufweisend:
Beleuchten mindestens eines Haars (102H) mit Licht, wobei das mindestens eine Haar (102H) derart entlang eines zylinderabschnittsähnlichen Trägers verlaufend angeordnet ist, dass eine Mehrzahl von nebeneinander angeordneten Haarabschnitten im Wesentlichen senkrecht zu einer Zylinderachse angeordnet ist;
wobei das Beleuchten mittels einer Lichtquelle (130) erfolgt, welche zusammen mit einer Kamera (102) und einer Datenverarbeitungsvorrichtung (104) Teil einer integrierten tragbaren Vorrichtung (100b) ist, wobei die integrierte tragbare Vorrichtung (100b) ein Smartphone, ein Tablet oder ein Phablet aufweist;
während des Beleuchtens, Aufnehmen eines digitalen Bildes (200) des mindestens einen Haars (102H) mittels der Kamera (102) der integrierten tragbaren Vorrichtung (100b);
Ermitteln eines Glanzwerts des Haars (102H) basierend auf dem digitalen Bild (200) mittels der Datenverarbeitungsvorrichtung (104) der integrierten tragbaren Vorrichtung (100b), wobei das Ermitteln des Glanzwerts ein Ermitteln eines maximalen Helligkeitswerts in einem Haarbereich des digitalen Bildes (200) und ein Zuordnen des maximalen Helligkeitswerts zu einem Glanzwert aufweist;
Ermitteln eines Haarpflegeprodukts zur Glanzerhöhung basierend auf dem ermittelten Glanzwert unter Verwendung einer vorgegebenen Zuordnung von Glanzwerten und geeigneten Haarpflegeprodukten;
Einleiten des Online-Bestellvorgangs für das ermittelte Haarpflegeprodukt als individuell hergestelltes Haarpflegeprodukt mittels der integrierten tragbaren Vorrichtung (100b),
wobei der Online-Bestellvorgang mit Hilfe eines Produktkonfigurators abläuft,
wobei der Produktkonfigurator einem Kunden bei einer Auswahl von Merkmalen und/oder Inhaltsstoffen des Haarpflegeprodukts hilft und auf zulässige und/oder unzulässige Merkmalskombinationen hinweist, wobei unzulässige Merkmalskombinationen nicht ausgewählt werden können, und
wobei der Produktkonfigurator die Auswahl für den ermittelten Glanzwert ungeeigneter Inhaltsstoffe vermeidet und/oder die Auswahl für den ermittelten Glanzwert geeigneter Inhaltsstoffe vorgibt oder vorschlägt.

2. Verfahren gemäß Anspruch 1,
wobei das Licht linear polarisiert ist, wobei eine Schwingungsebene des polarisierten Lichts entlang der Zylinderachse (ZA) verläuft.

3. Verfahren gemäß Anspruch 1 oder 2,
wobei das Ermitteln des Glanzwerts des Haars (102H) ferner ein Ermitteln eines Glanzbereichs (200G) in einem Haarbereich des digitalen Bildes (200), ein Summieren von Glanz-Helligkeitswerten aller Bildelemente im Glanzbereich (200G) und ein Zuordnen der Summe zu einem Glanzwert aufweist.

4. Verfahren gemäß einem der vorhergehenden Ansprüche, ferner aufweisend:
Anordnen der Kamera (102) derart, dass ein Winkel zwischen einer Hauptrichtung, aus der das Licht das Haar (102H) beleuchtet, und einer optischen Achse der Kamera (102) etwa 45° beträgt.

5. Verfahren gemäß einem der vorhergehenden Ansprüche, ferner aufweisend:
Anordnen des Haars (102H) auf einem zylinderartigen Körper (136).

6. Verfahren gemäß einem der vorhergehenden Ansprüche,
wobei das digitale Bild (200) in einem Farbraum parametrisiert ist, der eine Helligkeit als einen Parameter aufweist.

7. Verfahren gemäß einem der vorhergehenden Ansprüche, ferner aufweisend:
Bereitstellen eines Hinweises, wo das ermittelte Haarpflegeprodukt erhältlich ist.

8. Integrierte tragbare Vorrichtung (100b) ausgebildet zum Ermitteln eines Glanzwerts von Haaren (102H), zum Ermitteln eines Haarpflegeprodukts zur Glanzerhöhung und zum Einleiten eines Online-Bestellvorgangs für ein individuell hergestelltes Haarpflegeprodukt, wobei die integrierte tragbare Vorrichtung (100b) ein Smartphone, ein Tablet oder ein Phablet aufweist, die integrierte tragbare Vorrichtung (100b) aufweisend:
eine Lichtquelle (130) zum Beleuchten von mindestens einem Haar (102H) mit Licht, wobei das mindestens eine Haar (102H) derart entlang eines zylinderabschnittsähnlichen Trägers verlaufend anordenbar ist, dass eine Mehrzahl von nebeneinander angeordneten Haarabschnitten im Wesentlichen senkrecht zu einer Zylinderachse (ZA) angeordnet ist;
eine Kamera (102) zum Aufnehmen eines digitalen Bildes (200) des mindestens einen Haars (102H) während des Beleuchtens; und
eine elektronische Datenverarbeitungsvorrichtung (104), die eingerichtet ist,
einen Glanzwert des Haars (102H) basierend auf dem digitalen Bild (200) zu ermitteln, wobei das Ermitteln des Glanzwerts ein Ermitteln eines maximalen Helligkeitswerts in einem Haarbereich des digitalen Bildes (200) und ein Zuordnen des maximalen Helligkeitswerts zu einem Glanzwert aufweist,
ein Haarpflegeprodukt zur Glanzerhöhung basierend auf dem ermittelten Glanzwert unter Verwendung einer vorgegebenen Zuordnung von Glanzwerten und geeigneten Haarpflegeprodukten zu ermitteln, und
einen Online-Bestellvorgang für das ermittelte Haarpflegeprodukt als individuell hergestelltes Haarpflegeprodukt einzuleiten,
wobei der Online-Bestellvorgang mit Hilfe eines Produktkonfigurators abläuft,
wobei der Produktkonfigurator einem Kunden bei einer Auswahl von Merkmalen und/oder Inhaltsstoffen des Haarpflegeprodukts hilft und auf zulässige und/oder unzulässige Merkmalskombinationen hinweist, wobei unzulässige Merkmalskombinationen nicht ausgewählt werden können, und
wobei der Produktkonfigurator die Auswahl für den ermittelten Glanzwert ungeeigneter Inhaltsstoffe vermeidet und/oder die Auswahl für den ermittelten Glanzwert geeigneter Inhaltsstoffe vorgibt oder vorschlägt.

9. Integrierte tragbare Vorrichtung (100b) gemäß Anspruch 8,
wobei das von der Lichtquelle (130) abgestrahlte Licht linear polarisiertes Licht ist.

## Claims

1. A method for initiating an online ordering process for a custom-made hair care product, comprising:
illuminating at least one hair (102H) with light, wherein the at least one hair (102H) is arranged to extend along a cylindrical-section-like support in such a way that a plurality of adjacent hair sections are arranged substantially perpendicular to a cylindrical axis;
wherein the illumination is provided by a light source (130) which, together with a camera (102) and a data processing device (104), forms part of an integrated portable device (100b), wherein the integrated portable device (100b) comprises a smartphone, a tablet or a phablet;
during illumination, capturing a digital image (200) of the at least one hair (102H) by means of the camera (102) of the integrated portable device (100b);
determining a gloss value of the hair (102H) based on the digital image (200) by means of the data processing device (104) of the integrated portable device (100b), wherein the determination of the gloss value comprises determining a maximum brightness value in a hair region of the digital image (200) and assigning the maximum brightness value to a gloss value;
determining a hair care product for enhancing shine based on the determined shine value, using a predefined mapping of shine values to suitable hair care products;
initiating the online ordering process for the identified hair care product as a custom-made hair care product by means of the integrated portable device (100b),
wherein the online ordering process is carried out with the aid of a product configurator,
whereby the product configurator assists a customer in selecting characteristics and/or ingredients of the hair care product and indicates permissible and/or impermissible combinations of characteristics, whereby impermissible combinations of characteristics cannot be selected, and
wherein the product configurator avoids the selection of ingredients unsuitable for the determined gloss value and/or specifies or suggests the selection of ingredients suitable for the determined gloss value.

2. A method according to claim 1,
wherein the light is linearly polarised, with a plane of vibration of the polarised light extending along the cylindrical axis.

3. A method according to claim 1 or 2,
wherein determining the gloss value of the hair (102H) further comprises determining a gloss region (200G) within a hair region of the digital image (200), summing the gloss brightness values of all pixels in the gloss region (200G), and assigning the sum to a gloss value.

4. A method according to one of the preceding claims, further comprising:
positioning the camera (102) such that the angle between a main direction from which light illuminates the hair (102H) and an optical axis of the camera (102) is approximately 45°.

5. A method according to one of the preceding claims, further comprising:
positioning the hair (102H) on a cylindrical body.

6. A method according to one of the preceding claims,
wherein the digital image (200) is parameterised in a colour space comprising brightness as a parameter.

7. A method according to one of the preceding claims, further comprising:
providing information on where the identified hair care product is available.

8. Integrated portable device (100b) for determining a shine value of hair (102H), for identifying a hair care product to enhance shine, and for initiating an online ordering process for a custom-made hair care product, wherein the integrated portable device (100b) comprises a smartphone, a tablet or a phablet, the integrated portable device (100b) comprising:
a light source (130) for illuminating at least one hair (102H) with light, wherein the at least one hair (102H) can be arranged along a cylindrical-section-like support in such a way that a plurality of adjacent hair sections are arranged substantially perpendicular to a cylindrical axis;
a camera (102) for capturing a digital image (200) of the at least one hair (102H) whilst it is being illuminated; and
an electronic data processing device (104) configured to
determine a gloss value of the hair (102H) based on the digital image (200), wherein determining the gloss value comprises determining a maximum brightness value in a hair region of the digital image (200) and mapping the maximum brightness value to a gloss value,
determining a hair care product for enhancing shine based on the determined shine value, using a predefined mapping of shine values to suitable hair care products, and
initiating an online ordering process for the determined hair care product as a custom-made hair care product,
wherein the online ordering process is carried out with the aid of a product configurator,
whereby the product configurator assists a customer in selecting characteristics and/or ingredients of the hair care product and indicates permissible and/or impermissible combinations of characteristics, whereby impermissible combinations of characteristics cannot be selected, and
wherein the product configurator avoids the selection of ingredients unsuitable for the determined shine value and/or specifies or suggests the selection of ingredients suitable for the determined shine value.

9. Integrated portable device (100b) according to claim 8,
wherein the light emitted by the light source (130) is linearly polarised light.

## Revendications

1. Procédé permettant de lancer une procédure de commande en ligne pour un produit de soins capillaires fabriqué sur mesure, comprenant :
l'éclairage d'au moins un cheveu (102H) avec de la lumière, ledit au moins un cheveu (102H) étant disposé le long d'un support en forme de segment de cylindre de telle sorte qu'une pluralité de segments de cheveux disposés côte à côte soit orientée sensiblement perpendiculairement à un axe du cylindre ;
l'éclairage étant assuré par une source lumineuse (130) qui, conjointement avec une caméra (102) et un dispositif de traitement de données (104), fait partie d'un dispositif portable intégré (100b), ledit dispositif portable intégré (100b) comprenant un smartphone, une tablette ou un phablet ;
pendant l'éclairage, la capture d'une image numérique (200) dudit au moins un cheveu (102H) à l'aide de la caméra (102) du dispositif portable intégré (100b) ;
détermination d'une valeur de brillance du cheveu (102H) sur la base de l'image numérique (200) à l'aide du dispositif de traitement de données (104) du dispositif portable intégré (100b), la détermination de la valeur de brillance comprenant la détermination d'une valeur de luminosité maximale dans une zone du cheveu de l'image numérique (200) et l'association de cette valeur de luminosité maximale à une valeur de brillance ;
déterminer un produit de soin capillaire destiné à augmenter la brillance sur la base de la valeur de brillance déterminée, en utilisant une correspondance prédéfinie entre les valeurs de brillance et les produits de soin capillaire appropriés ;
le lancement de la procédure de commande en ligne du produit de soin capillaire déterminé, en tant que produit de soin capillaire fabriqué sur mesure, à l'aide du dispositif portable intégré (100b),
le processus de commande en ligne s'effectuant à l'aide d'un configurateur de produit,
le configurateur de produit aidant un client à sélectionner des caractéristiques et/ou des ingrédients du produit de soin capillaire et signalant les combinaisons de caractéristiques autorisées et/ou non autorisées, les combinaisons de caractéristiques non autorisées ne pouvant pas être sélectionnées, et
le configurateur de produit évitant la sélection d'ingrédients inadaptés à la valeur de brillance déterminée et/ou imposant ou proposant la sélection d'ingrédients adaptés à cette valeur de brillance.

2. Procédé selon la revendication 1,
dans lequel la lumière est polarisée linéairement, un plan d'oscillation de la lumière polarisée s'étendant le long de l'axe cylindrique.

3. Procédé selon la revendication 1 ou 2,
dans lequel la détermination de la valeur de brillance du cheveu (102H) comprend en outre la détermination d'une zone de brillance (200G) dans une partie du cheveu de l'image numérique (200), la sommation des valeurs de luminosité de brillance de tous les éléments d'image dans la zone de brillance (200G) et l'attribution de cette somme à une valeur de brillance.

4. Procédé selon l'une des revendications précédentes, comprenant en outre :
le positionnement de la caméra (102) de telle sorte que l'angle entre une direction principale à partir de laquelle la lumière éclaire les cheveux (102H) et un axe optique de la caméra (102) soit d'environ 45°.

5. Procédé selon l'une des revendications précédentes, comprenant en outre :
le positionnement du cheveu (102H) sur un corps de forme cylindrique.

6. Procédé selon l'une des revendications précédentes,
dans lequel l'image numérique (200) est paramétrée dans un espace colorimétrique comportant une luminosité comme paramètre.

7. Procédé selon l'une des revendications précédentes, comprenant en outre :
la fourniture d'une indication précisant où le produit de soins capillaires identifié est disponible.

8. Dispositif portable intégré (100b) destiné à déterminer un indice de brillance des cheveux (102H), pour déterminer un produit de soin capillaire destiné à augmenter la brillance et pour lancer une procédure de commande en ligne d'un produit de soin capillaire à fabriquer sur mesure, le dispositif portable intégré (100b) comprenant un smartphone, une tablette ou un phablet, le dispositif portable intégré (100b) comprenant :
une source lumineuse (130) destinée à éclairer au moins un cheveu (102H) avec de la lumière, ledit au moins un cheveu (102H) pouvant être disposé le long d'un support en forme de segment cylindrique de telle sorte qu'une pluralité de sections de cheveux disposées côte à côte soit orientée sensiblement perpendiculairement à un axe cylindrique ;
une caméra (102) destinée à capturer une image numérique (200) dudit au moins un cheveu (102H) pendant son éclairage ; et
un dispositif de traitement électronique des données (104) conçu pour
déterminer une valeur de brillance du cheveu (102H) sur la base de l'image numérique (200), la détermination de la valeur de brillance comprenant la détermination d'une valeur de luminosité maximale dans une zone du cheveu de l'image numérique (200) et l'association de cette valeur de luminosité maximale à une valeur de brillance,
déterminer un produit de soin capillaire destiné à augmenter la brillance sur la base de la valeur de brillance déterminée, en utilisant une correspondance prédéfinie entre les valeurs de brillance et les produits de soin capillaire appropriés, et
lancer une procédure de commande en ligne pour le produit de soin capillaire déterminé, en tant que produit de soin capillaire fabriqué sur mesure,
le processus de commande en ligne s'effectuant à l'aide d'un configurateur de produit,
le configurateur de produit aidant un client à sélectionner les caractéristiques et/ou les ingrédients du produit de soin capillaire et signalant les combinaisons de caractéristiques autorisées et/ou non autorisées, les combinaisons de caractéristiques non autorisées ne pouvant pas être sélectionnées, et
le configurateur de produit évitant la sélection d'ingrédients inadaptés à la valeur de brillance déterminée et/ou imposant ou proposant la sélection d'ingrédients adaptés à cette valeur de brillance.

9. Dispositif portable intégré (100b) selon la revendication 8,
dans lequel la lumière émise par la source lumineuse (130) est une lumière polarisée linéairement.
